# EUROPÄISCHE PATENTSCHRIFT

(11) **EP 4 197 494 B1**
(45) Veröffentlichungstag und Bekanntmachung des Hinweises auf die Patenterteilung: **05.02.2025**
(21) Anmeldenummer: 21215423.1
(22) Anmeldetag: 17.12.2021
(51) Int. Cl.: A61F 2/30, A61F 2/28

(54) **AUGMENTATIONSVORRICHTUNG, KOMPOSIT UND VERFAHREN ZUR HERSTELLUNG EINES KOMPOSITS**
AUGMENTATION DEVICE, COMPOSITE AND METHOD FOR MAKING A COMPOSITE
DISPOSITIF D'AUGMENTATION, COMPOSITE ET PROCÉDÉ DE FABRICATION D'UN COMPOSITE

(43) Veröffentlichungstag der Anmeldung: 21.06.2023
(73) Patentinhaber: Heraeus Medical GmbH, 61273 Wehrheim (DE)
(72) Erfinder: VOGT, Sebastian, 63450 Hanau (DE); KLUGE, Thomas, 63450 Hanau (DE)
(74) Vertreter: Heraeus IP

(56) Entgegenhaltungen:
- FR-A1- 2 712 486
- US-A1- 2004 249 464
- US-A1- 2006 265 077
- US-B2- 9 943 411

## Beschreibung

Die Erfindung betrifft ein Augmentationsmaterial umfassend einen Draht und in axialer Ausrichtung entlang des Drahtes eine Vielzahl an Gruppen von sich radial aus dem Draht erstreckenden und axial benachbarten Verbindungselementen, wobei die Verbindungselemente derart ausgestaltet sind, dass beim Aneinanderpressen einer ersten Gruppe aus der Vielzahl an Gruppen gegen eine weitere Gruppe aus der Vielzahl an Gruppen die Verbindungselement der beiden Gruppen form- und/oder kraftschlüssig miteinander verbindbar sind.

Die Erfindung betrifft weiterhin ein Komposit umfassend das Augmentationsmaterial und einen Knochenzement sowie ein Verfahren zur Herstellung eines Komposits.

Gegenstand der Erfindung ist insbesondere eine alloplastisches Augmentationsmaterial zum Einsatz bei Gelenkendoprothesenoperationen, insbesondere Revisionsgelenkendoprothesenoperationen.

Das erfindungsgemäße Augmentationsmaterial ist zum Auffüllen und Stabilisieren von Kavitäten, insbesondere von Knochenkavitäten, wie beispielsweise einem Knochenkanal, bestimmt. Dazu kann das Augmentationsmaterial als Komposit mit einem Knochenzement ausgeformt werden.

### Hintergrund der Erfindung

Augmentationsmaterialien sind seit langem bekannt und werden umfangreich klinisch genutzt (J. M. Rueger: Knochenersatzmittel, Orthopäde 27 (1998) 72-79.). Die bisher verwendeten Augmentationsmaterialien sind im Allgemeinen volumenstabil, aber nicht formstabil. Eine Ausnahme bildet ein Augmentationsmaterial, das unter der Bezeichnung "Trabecular metal^{™}" von der Firma Zimmer vertrieben wird und das beispielsweise aus der WO 2013/074 909 A1 bekannt ist. Dieses Material hat eine poröse Struktur, die der Struktur der humanen Spongiosa (Schwammgewebe) nachgebildet ist. Dieses Material umfasst Tantal und ist in definierten Formen und Größen handelsüblich. Das Material ist im Operationssaal nicht in seiner Form und Größe veränderbar und kann nicht mit konventionellen Werkzeugen im Operationssaal bearbeitet werden. Die jeweilige anatomische Situation des Patienten kann daher nur bedingt berücksichtigt werden. Der medizinische Anwender kann nur versuchen, dass Implantatlager des Patienten an die vorgegebene Geometrie anzupassen oder ein annähernd passendes Implantat einzusetzend und die bestehenden Lücken mit allogenem Knochenmaterial oder anderem Volumenfüllern zu schließen.

In der Patentschrift EP 3 092 076 B1 wird ein partikuläres alloplastisches Augmentationsmaterial zur Herstellung eines frei geformten porösen Körpers beschrieben. Dazu greifen die einzelnen Partikel des Augmentationsmaterials beim Zusammenpressen der Partikel derart ineinander, dass ein frei formbarer poröser Körper entsteht, welcher anschließend mit einem Knochenzement befüllt werden kann, um ein Komposit herzustellen. Nachteilig gestaltet sich bei einem partikulären Augmentationsmaterial, dass dieses im Zuge einer Operation unsicher in der Anwendung ist. Beispielsweise muss darauf geachtet werden, dass die Partikel nicht unbeabsichtigt an ungewünschte Stellen im Patientenkörper gelangen, was den Operationsablauf behindern oder im schlimmsten Fall zu gesundheitlichen Komplikationen beim Patienten führen kann. Zudem ist die Ausformung des frei geformten porösen Körpers aus einem partikulären Material zeitintensiv, was insbesondere in zeitkritischen Operationen nachteilig ist. Lose Partikel, insbesondere lose auf den Operationssaalboden gefallene, Partikel stellen zudem ein nicht unerhebliches Unfallpotential dar.

In der Patentschrift US 9 943 411 B2 wird ein Augmentationsmaterial offenbart, welches einen Draht umfasst und in axialer Ausrichtung entlang einer Längsachse des Drahtes eine Vielzahl an Gruppen von sich radial aus dem Draht erstreckenden und axial benachbarten Verbindungselementen, wobei die Verbindungselemente derart ausgestaltet sind, dass beim Gegeneinanderpressen einer ersten Gruppe aus der Vielzahl an Gruppen gegen eine weitere Gruppe aus der Vielzahl an Gruppen die Verbindungselemente der beiden Gruppen form- und/oder kraftschlüssig miteinander verbindbar sind.

### Aufgaben

Eine Aufgabe der vorliegenden Erfindung ist es, einen oder mehrere der sich aus dem Stand der Technik ergebenen Nachteile zumindest teilweise zu überwinden.

Insbesondere soll ein Augmentationsmaterial bereitgestellt werden, welches einfach, schnell und anwendungssicher frei geformt werden kann und zur Auffüllung von Knochenkavitäten geeignet ist. Dabei soll das Augmentationsmaterial der Kontur der Kavität folgen können. Das Augmentationsmaterial soll nach dem Ausformen einen stabilen porösen Körper bildet, ohne dass chemische Aushärtungsreaktionen, wie beispielsweise radikalische Polymerisationen, notwendig sind. Das Knochenersatzmaterial soll nach der Formung eine offene Porosität besitzen und mechanisch stabil sein. Die Porosität und die Größe der Poren sollen dabei dazu ausreichen und dazu geeignet sein, dass menschlicher Knochen eines Patienten, der mit dem Knochenersatzmaterial behandelt wird, in die Poren einwachsen kann. Weiterhin sollen die Poren geeignet sein durch Infiltration mit einem Knochenzementteig, insbesondere mit einem PMMA-Knochenzementteig, stabilisiert zu werden. Das Augmentationsmaterial soll mit dem Knochenzement einen druckstabilen Komposit ausbilden können. Dabei soll eine Überhitzung des Komposits während der Aushärtung des Knochenzementteigs, insbesondere des PMMA-Knochenzementteigs, unter Schädigung des Gewebes des Patienten vermieden wird. Das Volumen des Augmentationsmaterials soll mit in Operationssälen verfügbaren Werkzeugen, wie beispielsweise Scheren oder Zangen, bearbeitet werden können.

Es ist eine weitere Aufgabe der Erfindung ein Komposit zur Verfügung zu stellen, welches einfach, schnell und frei formbar unter Verwendung des Augmentationsmaterials hergestellt werden kann und Beschränkungen herkömmlicher Komposite vermeidet.

Ein weiteres Ziel der Erfindung ist es, ein Verfahren zur Herstellung eines Komposits zur Verfügung zu stellen, welches Beschränkungen herkömmlicher Verfahren zur Herstellung eines Komposits vermeidet.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche 1 und 8 geleistet, durch welche die Erfindung definiert ist. Die abhängigen Ansprüche stellen bevorzugte Ausführungsformen bereit, die zur mindestens teilweisen Erfüllung mindestens einer der Aufgaben beitragen.

Die Erfindung umfasst ein Augmentationsmaterial umfassend einen Draht und in axialer Ausrichtung entlang einer Längsachse des Drahtes eine Vielzahl an Gruppen von sich radial aus dem Draht erstreckenden und axial benachbarten Verbindungselementen, wobei die Verbindungselemente derart ausgestaltet sind, dass beim Gegeneinanderpressen einer ersten Gruppe aus der Vielzahl an Gruppen gegen eine weitere Gruppe aus der Vielzahl an Gruppen die Verbindungselemente der beiden Gruppen form- und/oder kraftschlüssig miteinander verbindbar sind, wobei die Verbindungselemente als sich radial aus dem Draht erstreckende Scheiben ausgebildet sind.

In einer Ausführungsform des Augmentationsmaterials weisen axial benachbarte Verbindungselemente in einer Gruppe einen axialen Verbindungselementabstand zueinander auf, welcher zumindest einer axialen Verbindungselementerstreckung eines Verbindungselements entlang der Längsachse des Drahtes entspricht.

In einer Ausführungsform des Augmentationsmaterials sind die Scheiben perforiert, weisen die Scheiben eine offenporige Struktur auf oder sind die Scheiben perforiert und weisen eine offenporige Struktur auf.

In einer Ausführungsform des Augmentationsmaterials umfasst eine Gruppe von Verbindungselementen 3 bis 20, bevorzugt 3 bis 15, weiter bevorzugt 5 bis 15 axial benachbarter Verbindungselemente.

In einer Ausführungsform des Augmentationsmaterials weisen axial benachbarte, insbesondere direkt axial benachbarte, Gruppen von Verbindungselementen einen axialen Gruppenabstand zueinander auf, welcher zumindest dem zweifachen der axialen Erstreckung eines Verbindungselements entlang der Längsachse des Drahtes entspricht.

Das Augmentationsmaterial kann mit einem generativen 3D-Druckverfahren hergestellt werden.

Eine weitere Ausführungsform der Erfindung ist ein Komposit umfassend ein Augmentationsmaterial nach einer der vorhergehenden Ausführungsformen der Erfindung und einen Knochenzement, insbesondere einen PMMA-Knochenzement, wobei das Augmentationsmaterial von dem Knochenzement, insbesondere von dem PMMA-Knochenzemente, umschlossen, insbesondere vollständig umschlossen, ist.

In einer Ausführungsform des Komposits nimmt das Augmentationsmaterial im Komposit einen Volumenanteil im Bereich von 30-70 Volumenprozent bezogen auf das Volumen des Komposits ein.

Eine weitere Ausführungsform der Erfindung ist ein Verfahren zur Herstellung eines Komposits, insbesondere eines Komposits nach der zwölften oder dreizehnten Ausführungsform der Erfindung, zum Befüllen einer Kavität, insbesondere einer Kavität in einem Knochen, insbesondere eines Knochenkanals, umfassend die Schritte
a. Bereitstellen des Augmentationsmaterials in einer Anordnung, welche im Wesentlichen der Form der Kavität entspricht;
b. Applizieren eines Knochenzementteigs in Zwischenräume des Augmentationsmaterials, so dass das Augmentationsmaterial vollständig von dem Knochenzementteig umschlossen ist; und
c. Aushärten des Knochenzementteigs unter Ausbildung des Komposits.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann.

Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen senkrechte Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

### Ausführliche Beschreibung

Der Gegenstand der Erfindung betrifft ein Augmentationsmaterial umfassend einen Draht und in axialer Ausrichtung entlang einer Längsachse des Drahtes eine Vielzahl an Gruppen von sich radial aus dem Draht erstreckenden und axial benachbarten Verbindungselementen, wobei die Verbindungselement derart ausgestaltet sind, dass beim Gegeneinanderpressen einer ersten Gruppe aus der Vielzahl an Gruppen gegen eine weitere Gruppe aus der Vielzahl an Gruppen die Verbindungselemente der beiden Gruppen, also der ersten Gruppe und der weiteren Gruppe aus der Vielzahl der Gruppen, formschlüssig oder kraftschlüssig oder form- und kraftschlüssig miteinander verbindbar sind, wobei die Verbindungselemente als sich radial aus dem Draht erstreckende Scheiben ausgebildet sind.

Das Augmentationsmaterial umfasst einen Draht. Unter einem Draht ist ein plastisch verformbarer, länglicher Körper zu verstehen. Der Draht kann einteilig ausgeformt sein oder auch aus mehreren, wie beispielsweise zwei bis zehn, Drahtteilelementen, optional mit einer oder mehreren zusätzlichen Einlagen, zusammengesetzt sein. Beispielsweise kann der Draht aus mehreren Drahtteilelemente, welche verdreht und/oder verflochten sind, zusammengesetzt sein. Aufgrund der einfachen Herstellung ist ein einteiliger Draht bevorzugt.

Der Draht kann unterschiedliche Querschnittsgeometrien, wie beispielsweise eckig, insbesondere vier- oder sechseckig, oval oder sternförmig aufweisen, wobei runde Querschnittsgeometrien aufgrund der einfacheren Herstellungsweise bevorzugt sind.

Der Drahtdurchmesser des Drahtes liegt vorzugsweise im Bereich von 0,1 mm bis 2 mm, weiter bevorzugt im Bereich von 0,2 mm bis 1,5 mm. Dies erlaubt einerseits ein einfaches Verformen des Drahtes, und damit des Augmentationsmaterials, in die gewünschte Form und erhält andererseits eine gewisse Stabilität des so aus dem Augmentationsmaterials geformten porösen Körpers. Zudem erlaubt ein derartiger Drahtdurchmesser ein einfaches Kürzen des Drahtes, und damit des Augmentationsmaterials, mit in einem Operationssaal vorhandenen Werkzeugen, wie beispielsweise einer Zange oder einer Schere.

Der Draht weist vorzugsweise eine Drahtlänge auf, welche im Bereich von 10 cm bis 50 cm, weiter bevorzugt im Bereich 10 cm bis 40 cm, auf. Dies erlaubt ein Auffüllen gängiger Kavitätengrößen im Zuge einer Operation.

Aus dem Draht erstrecken sich radial, in einem Winkel in einem Bereich von 60°-120° zur Längsachse des Drahtes, eine Vielzahl an Gruppen von axial benachbarten Verbindungselementen.

Unter einem Verbindungselement ist eine strukturelle Erhebung an oder auf der Mantelfläche des Drahtes zu verstehen. In einer Ausgestaltungsform sind der Draht und die Verbindungselemente form- und/oder kraftschlüssig miteinander verbunden. In einer weiteren Ausgestaltungsform sind der Draht und die Verbindungselemente stoffschlüssig miteinander verbunden. In einer weiteren, bevorzugten Ausgestaltungsform sind der Draht und die Verbindungselemente einteilig ausgeformt, wobei die Mantelfläche des Drahtes selbst als die Vielzahl an Verbindungselementen ausgeformt ist. Letzteres ist aufgrund der einfacheren und kostengünstigeren Herstellungsweise bevorzugt.

Verbindungselemente sind axial benachbart, wenn sie an oder auf unterschiedlichen Positionen entlang der Längsachse des Drahtes angeordnet sind.

Die Verbindungselemente sind in einer Vielzahl an Gruppen, zumindest also zwei Gruppen, angeordnet, wobei eine Gruppe zumindest eine Vielzahl, zumindest also zwei, an axial, entlang der Längsachse des Drahtes angeordneten, benachbarten Verbindungselemente aufweist. Die Vielzahl an Gruppen von Verbindungselementen kann dabei derart zueinander angeordnet sein, dass diese wie eine einzige große Gruppe von Verbindungselementen erscheint.

Die Verbindungselemente sind dabei so ausgeformt, dass ein Gegeneinanderpressen zweier Gruppen von Verbindungselementen zu einem Formschluss, zu einem Kraftschluss oder zu einem Form- und Kraftschluss zwischen den Verbindungselementen der beiden Gruppen kommt. Die Verbindungselemente der Gruppen greifen dabei ineinander, um einen Form- und/oder Kraftschluss auszubilden. Das Gegeneinanderpressen zweier Gruppen von Verbindungselementen kann beispielsweise durch ein Verformen des Drahtes ermöglicht werden. Beispielsweise kann der Draht gebogen oder gewickelt werden, so dass die beiden Gruppen von Verbindungselementen an den beiden axial entgegengesetzten Enden des Drahtes, und damit des Augmentationsmaterials, miteinander verbunden werden. In einer Ausgestaltungsform ist die Verbindung der Gruppen reversibel, so dass diese wieder voneinander lösbar sind. In einer weiteren Ausgestaltungsform ist die Verbindung irreversibel, so dass diese nicht zerstörungsfrei wieder gelöst werden kann.

Durch den flexiblen Draht kann das Augmentationsmaterial schnell, einfach und anwendungssicher in beliebig geformte poröse Körper, insbesondere zur Auffüllung von Knochenkavitäten, gebracht werden. Die Verbindungselemente ermöglichen dabei eine Stabilisierung des derart geformten porösen Körpers, ohne dass sich das Augmentationsmaterial selbstständig wieder in seine ursprüngliche Form verbringt. Das so in Form gebrachte Augmentationsmaterial bildet einen porösen Körper, wobei die Poren des porösen Körpers die Zwischenräume der Verbindungselemente und des in Form gebrachten Drahtes sind.

Da das Augmentationsmaterial einstückig ausgebildet ist, kann es anwendungssicher im Zuge einer Operation verwendet werden.

Um das Augmentationsmaterials an die Größe der zu befüllenden Kavität anzupassen, kann der Draht an beliebiger Stelle, insbesondere zwischen axial benachbarten Verbindungselementen, gekürzt werden. In Abhängigkeit des für den Draht verwendeten Materials kann dies beispielsweise unter Verwendung einer Schere oder einer Zange erfolgen.

Der axiale Verbindungselementabstand, also der axiale Abstand zweier axial benachbarter Verbindungselemente in einer Gruppe, kann unterschiedliche Werte annehmen. Aufgrund der einfacheren Herstellungsweise weisen alle Verbindungselemente in einer Gruppe den gleichen axialen Verbindungselementabstand auf.

In einer Ausgestaltungsform des Augmentationsmaterials weisen axial benachbarte Verbindungselemente in einer Gruppe einen axialen Verbindungselementabstand zueinander auf, welcher zumindest einer axialen Verbindungselementerstreckung eines Verbindungselements entlang der Längsachse des Drahtes entspricht. Unter dem axialen Verbindungselementabstand ist der parallel zu Längsachse des Drahtes verlaufende Abstand zweier Verbindungselemente auf Höhe des Drahtes zu verstehen.

Die Verbindungselemente können über deren radiale, im Wesentlichen senkrecht zur Längsachse des Drahtes verlaufenden, Erstreckung ungleichmäßig dick ausgeformt sein. Beispielsweise können die Verbindungselemente direkt am Draht anliegend eine axiale Erstreckung aufweisen, welche mit sich mit zunehmendem radialem Abstand zum Draht verringert. Beispielsweise ist dies bei Verbindungselementen der Fall, welche als konzentrisch um den Draht ausgeformte, diskusartigen Scheiben ausgebildet sind. Unter der axialen Verbindungselementerstreckung ist dabei die axiale Erstreckung des Teils des Verbindungselements mit dem größten radialen Abstand zum Draht zu verstehen.

Vorzugsweise weist der axiale Verbindungselementabstand einen Wert auf, welcher in einem Bereich des 1- bis 2-fachen, vorzugweise des 1,05- bis 1,8-fachen, des Wertes der axialen Verbindungselementerstreckung liegt. Dies ermöglicht ein einfaches Einschieben eines Verbindungselements einer ersten Gruppe zwischen zwei axial benachbarte Verbindungselemente einer weiteren Gruppe von Verbindungselementen unter Ausbildung eines Form- und/oder Kraftschlusses zwischen den Verbindungselementen. Insbesondere eine passgenaue formschlüssige Verbindung zwischen gegeneinandergepressten Verbindungselementen kann beispielsweise dann ausgebildet werden, wenn der axiale Verbindungselementabstand der axialen Verbindungselementerstreckung entspricht. Weist der axiale Verbindungselementabstand einen kleineren Wert als das 1-fache der axialen Verbindungselementerstreckung auf, wird das Ausbilden eines Form- und/oder Kraftschlusses zwischen den gegeneinandergepressten Verbindungselementen erschwert. Weist der axiale Verbindungselementabstand einen größeren Wert als das 2-fache der axialen Verbindungselementerstreckung auf, verringert sich insbesondere die Haltekraft des Kraftschlusses der gegeneinandergepressten Verbindungselemente und der aus dem Augmentationsmaterial geformte poröse Körper verliert an Stabilität.

Vorzugsweise weisen die Verbindungselemente eine axiale Verbindungselementerstreckung in einem Bereich von 0,05 mm bis 1,5 mm, vorzugsweise in einem Bereich von 0,5 bis 1 mm, auf, um eine gute Handhabung des Augmentationsmaterials zu gewährleisten.

In einer Ausgestaltungsform des Augmentationsmaterials sind die Verbindungselemente als sich radial aus dem Draht erstreckende Scheiben ausgebildet. Aufgrund der einfachen Herstellungsweise und der leichteren Anwendbarkeit ist es bevorzugt, dass die Scheiben im Wesentlichen konzentrisch um den Draht angeordnet sind. Die scheibenförmigen Verbindungselemente erlauben ein Gegeneinanderpressen über den vollen radialen Umfang des Drahtes, so dass eine hohe Variation von geformten porösen Körpern mittels des Augmentationsmaterials ohne Schwierigkeiten zugänglich ist.

Die Scheiben können unterschiedlich geformte Querschnittsgeometrien aufweisen.

Beispielsweise können die Scheiben einen elliptischen, eckigen, insbesondere vier-, fünf- oder sechseckigen aufweisen, wobei Scheiben mit einer runden Querschnittsgeometrie aufgrund der einfacheren Herstellungsweise bevorzugt sind.

Die als Scheiben ausgebildeten Verbindungselemente können eine geschlossene, glatte Oberflächenstruktur aufweisen.

In einer Ausgestaltungsform des Augmentationsmaterials sind die als Scheiben ausgeformten Verbindungselemente perforiert, und weisen somit ein oder mehrere Durchführungen in axialer Richtung entlang der Längsachse des Drahtes auf. In einer Ausgestaltungsform weisen die als Scheiben ausgeformte Verbindungselemente eine offenporige Struktur auf. In einer Ausgestaltungsform sind die als Scheiben ausgeformte Verbindungselement perforiert und weisen eine offenporige, schwammartige Struktur auf. Die vorgenannten Ausgestaltungsformen erlauben ein verbessertes Einwachsen von sich neubildendem Knochengewebe, sowohl in die Zwischenräume des geformten porösen Körpers, und somit in die Zwischenräume zwischen dem Draht und den Scheiben, als auch in die Perforationen und/oder in die offenporöse Struktur der einzelnen Scheiben. Zudem wird eine Verzahnung mit Knochenzement durch derartige Verbindungselemente verbessert.

In einer nicht erfindungsgemäßen, beispielhaften Ausgestaltungsform des Augmentationsmaterials sind die Verbindungselemente als sich radial aus dem Draht erstreckende Stifte ausgebildet. Unter Stiften sich längliche Struktureinheiten zu verstehen, deren Längsachse einen Winkel mit der Längsachse des Drahtes im Bereich von 60° bis 120° einschließt. Aufgrund der einfachen Herstellung ist es bevorzugt, wenn die Längsachse der Stifte und die Längsachse des Drahtes im Wesentlichen senkrecht zueinanderstehen. Dabei ist es bevorzugt, wenn alle Stifte eine gleiche Stiftlänge und einen gleichen Stiftdurchmesser aufweisen.

Die Stifte können derart auf oder an dem Draht angeordnet sein, dass jeder Stift einen oder zwei axial benachbarte Stifte aufweist, so dass an jeder gegebenen Querschnittsebenen des Drahtes maximal ein Stift angeordnet ist. Beispielsweise können dabei alle Stifte in die vom Draht aus gesehen gleiche Richtung weisen, wie dies beispielsweise bei Zinken eines Kamms ausgehend von dessen Griff der Fall ist.

In einer nicht erfindungsgemäßen, beispielhaften Ausgestaltungsform erstrecken sich mehrere, insbesondere 4 bis 10, bevorzugt 6 bis 8.

Stifte radial benachbart aus dem Draht. In den entsprechenden Querschnittsebenen des Drahtes sind somit mehrere, insbesondere 4 bis 10, bevorzugt 6 bis 8, Stifte angeordnet. Die radial benachbarten Stifte erstrecken sich scheibenartig oder sternförmig im Wesentlichen in einer Ebene aus dem Draht. Dies ermöglicht eine verbesserte form- und/oder kraftschlüssige Verbindung gegeneinandergepresster Stifte verschiedener Gruppen an Verbindungselementen untereinander. Insbesondere lassen sich die Gruppen an Verbindungselementen aus variablen Richtungen und Winkeln zueinander form- und/oder kraftschlüssig miteinander verbinden, so dass der Anwender des Augmentationsmaterials freier in seiner Ausgestaltung des aus dem Augmentationsmaterials geformten porösen Körpers ist.

Die Stifte können unterschiedlich lang ausgeformt sein, um eine form- und/oder kraftschlüssige Verbindung untereinander auszuformen.

In einer nicht erfindungsgemäßen, beispielhaften Ausgestaltungsform des Augmentationsmaterials erstrecken sich die Stifte mit einer Stiftlänge radial aus dem Draht, welche zumindest dem dreifachen des Drahtdurchmessers des Drahtes entspricht. Dies erlaubt die Ausbildung eines möglichst stabilen porösen Körpers aus dem Augmentationsmaterial. Um die Handhabung des Augmentationsmaterials nicht zu erschweren, ist es bevorzugt, wenn die Stiftlänge nicht mehr als dem zehnfachen des Drahtdurchmessers entspricht.

Die Stifte können unterschiedliche Querschnittsgeometrien wie beispielsweise eckig, insbesondere vier-, fünf- oder sechseckig, oval oder rund aufweisen, wobei eine runde Querschnittsgeometrie aufgrund der einfacheren Herstellungsweise bevorzugt ist. Insbesondere können die Stifte zylinderförmig ausgeformt sein, wobei das dem Draht abgewandte Stiftende kantig oder rund, insbesondere halbkugelförmig, ausgestaltet sein kann.

Um die form- und/oder kraftschlüssige Verbindung der Stifte beim Gegeneinanderpressen zweiter Gruppen von Stiften weiter zu verbessern, umfassen die Stifte in einer nicht erfindungsgemäßen, beispielhaften Ausgestaltungsform des Augmentationsmaterials Pilze, also pilzförmige, im Wesentlichen halbkugelförmige, über den eigentlichen Stiftdurchmesser überstehende Struktureinheiten, Haken, Schlaufen, Hinterschnitte und/oder Rastelemente auf oder sind die Stifte als Pilze, Haken, Schlaufen, Hinterschnitte und/oder Rastelemente ausgebildet.

Beispielsweise können die Stifte mit Pilzen, also pilzförmigen Struktureinheiten, insbesondere an dem dem Draht entgegengesetzten Stiftende, versehen sein, so dass die Pilze der aus unterschiedlichen Gruppen gegeneinandergepressten Stifte miteinander widerhakenartig miteinander rasten und so die Verbindung der Stifte stabiler ausformen als dies ohne Pilze der Fall wäre.

In einer Ausgestaltungsform des Augmentationsmaterials umfasst eine Gruppe von Verbindungselementen 3 bis 20, bevorzugt 3 bis 15, weiter bevorzugt 5 bis 15 axial benachbarte Verbindungselemente.

Beispielsweise weist eine Gruppe von Verbindungselementen 10 axial benachbarte Scheiben auf. Neben den axial benachbarten Verbindungselementen, insbesondere bei Verbindungselementen in Form von nicht erfindungsgemäßen Stiften, können die axial benachbarten Verbindungselemente zusätzliche radial benachbarte Verbindungselemente aufweisen. Beispielsweise kann eine Gruppe 10 axial benachbarter Stifte umfassen, wobei jeder dieser 10 axial benachbarter Stifte jeweils weitere 5 radial benachbarte Stifte aufweist, welcher jeweils im Wesentlichen in der gleichen Querschnittsebene des Drahtes liegen wie die entsprechenden axial benachbarten Stifte. Eine derartige Gruppe von Verbindungselementen umfasst damit insgesamt 60 Stifte.

Die Gruppen von Verbindungselementen können so dicht axial beieinander angeordnet sein, dass sie wie eine große Gruppe von Verbindungselementen erscheinen.

In einer Ausgestaltungsform des Augmentationsmaterials weisen axial benachbarte Gruppen von Verbindungselementen einen axialen Gruppenabstand zueinander auf, welcher zumindest dem zweifachen der axialen Erstreckung eines Verbindungselements entlang der Längsachse des Drahtes entspricht. Unter dem axialen Gruppenabstand ist der parallel zu Längsachse des Drahtes verlaufende Abstand zweier Gruppen von Verbindungselementen zu verstehen.

Dies ermöglicht eine verbesserte Biegsamkeit des Drahtes und sorgt gleichzeitig dafür, dass der aus dem Augmentationsmaterial geformte poröse Körper eine höhere Anzahl sowie größere Poren aufweist, was ein verbessertes Einwachsen von Knochengewebe in den porösen Körper und/oder ein verbessertes Eindringen von Knochenzementteig in den porösen Körper erlaubt.

Das Augmentationsmaterial kann auf unterschiedliche Weisen hergestellt werden.

In einer Ausgestaltungsform des Augmentationsmaterials kann das Augmentationsmaterial mit einem generativen 3D-Druckverfahren hergestellt werden.

Ein, insbesondere bevorzugtes, Beispiel für ein 3D-Druckverfahren ist das selektive Laserschmelzen SLM (Selective Laser Melting). Andere Rapid-Prototyping-Verfahren beziehungsweise computergestützte generative Herstellungsverfahren können auch zur Herstellung des Augmentationsmaterials verwendet werden, wie beispielsweise Fused Layer Modeling/Manufacturing (FLM), Fused Deposition Modeling (FDM), Laminated Object Modelling (LOM) von Kunststofffolien, Layer Laminated Manufacturing (LLM) von Kunststofffolien, Elektronenstrahlschmelzen (EBM) von Kunststoffen oder Metallen, Multi Jet Modeling (MJM) von Kunststoffen, Selektives Lasersintern (SLS) von Kunststoffen oder Metallen, und Stereolithografie (STL oder SLA).

Das Augmentationsmaterial kann unterschiedliche Materialien umfassen oder aus unterschiedlichen Materialien bestehen. Beispielsweise kann das Augmentationsmaterial aus biokompatiblem Kunststoff, Edelstahl, Titan, einer Titanlegierung, Tantal, einer Tantallegierung oder aus Verbundstoffen dieser Materialien gebildet sein.

Diese Materialien sind für medizinische Zwecke besonders gut einsetzbar und mit diesen Materialien lassen sich auch geeignete elastischen Eigenschaften für die Verbindungselemente einstellen. Aus Metall oder Metalllegierungen bestehende Augmentationsmaterialien können bevorzugt durch selektives Lasersintern oder auch durch Schmelzen mit Elektronenstrahlen hergestellt werden, bevorzugt mit einem 3D-Druckverfahren.

Der biokompatible Kunststoff kann biodegradierbar sein. Dazu können Polylactide, Polylglycolide, Polycaprolactone und Polyester verwendet werden, die aus unterschiedlichen a-Hydroxycarbonsäuren gebildet sind. Als nicht biodegradierbare Kunststoffe kommen Polyamide, Polyimide, Polyetherketon und Polysulfon in Betracht. Augmentationsmaterialien aus diesen nicht biodegradierbaren und degradierbaren Kunststoffen können durch selektives Lasersintern hergestellt werden.

Ein weiterer Gegenstand der Erfindung betrifft ein Komposit umfassend ein Augmentationsmaterial nach einer der vorhergehenden Ausgestaltungsformen und einen Knochenzement, insbesondere einen PMMA-Knochenzement, wobei das Augmentationsmaterial von dem Knochenzement umschlossen, insbesondere vollständig umschlossen, ist.

Zur Herstellung des Komposits wird das Augmentationsmaterial von einem Knochenzementteig umschlossen, insbesondere vollständig umschlossen, welcher im Anschluss zu einem Knochenzement aushärtet.

Unter einem Knochenzementteig wird eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen und/oder Wirbelkörper zu stabilisieren. Durch Aushärtung wird aus einem Knochenzementteig ein Knochenzement. Bevorzugt handelt es sich bei diesen Knochenzementen um Polymethylmethacrylat-Knochenzemente (PMMA-Knochenzemente) oder um anorganische Knochenzemente. PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einer Pulverkomponente umfassend ein Knochenzementpulver als erster Ausgangskomponente und einer Flüssigkeitskomponente umfassend eine Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzementteig. Dabei wird eine Polymerisation des Monomers durch Radikale eingeleitet. Mit fortschreitender Polymerisation des Monomers erhöht sich die Viskosität des Knochenzementteigs, bis dieser vollständig aushärtet.

Unter einem Knochenzementpulver wird ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulver zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver als hydrophiles Additiv mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Die Partikelgröße des partikulären Polymethylmethacrylat und/oder des partikulären Polymethylmethacrylat-Copolymers des Knochenzementpulvers der Siebfraktion kann kleiner 150 µm, bevorzugt kleiner 100 µm, sein. Das hydrophile Additiv kann partikulär und/oder faserförmig ausgestaltet sein.

In einer weiteren, nicht erfindungsgemäßen Ausgestaltungsform kann das hydrophile Additiv schwerlöslich, bevorzugt unlöslich, in Methylmethacrylat sein. In einer weiteren, nicht erfindungsgemäßen Ausgestaltungsform kann das hydrophile Additiv ein Aufsaugvermögen von mindestens 0,6 g Methylmethacrylat pro Gramm hydrophiles Additiv besitzen. In einer weiteren, nicht erfindungsgemäßen Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer nicht erfindungsgemäßen

Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Der Initiator kann Dibenzoylperoxid enthalten oder aus Dibenzoylperoxid bestehen.

Man versteht unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen.

Die pharmazeutisch aktive Substanz kann ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin.

Die Monomerflüssigkeit kann das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin. Unter einem anorganischen Knochenzement wird ein Knochenzement auf Basis von Calciumphosphaten und Calciumsulfat-Dihydrat verstanden. Als Pulverkomponente kommen dabei Pulver von Calciumphosphaten und/oder Calciumsulfat-Dihydrat zum Einsatz, welche durch eine Flüssigkeitskomponente umfassend eine wässrige Lösung verschiedener Salze aushärtbar ist. Es wurde eine Vielzahl an anorganischen Knochenzementen beschrieben, von denen exemplarisch folgende genannt sind: EP 1 592 463 B1, EP 2 271 585 B1 und EP 2 988 789 B1.

Der Knochenzement füllt insbesondere Zwischenräume des aus dem Augmentationsmaterial geformten porösen Körpers.

Das Komposit bildet einen druckstabilen, nicht-porösen Körper, in den kein Knochengewebe einwachsen kann.

Das Komposit kann unterschiedlichen Volumenanteile von Augmentationsmaterial zu Knochenzement aufweisen.

In einer Ausgestaltungsform des Komposits nimmt das Augmentationsmaterial im Komposit einen Volumenanteil im Bereich von 30 Volumenprozent bis 70 Volumenprozent, vorzugsweise einen Volumenanteil von 35 Volumenprozent bis 65 Volumenprozent, bezogen auf das Volumen des Komposits ein. Durch den Volumenanteil von mindestens 30 Volumenprozent an Augmentationsmaterial kann das Komposit mittels eines Knochenzementteigs, insbesondere eines PMMA-Knochenzementteigs, hergestellt werden, ohne dass es beim Aushärten des Knochenzementteigs, insbesondere des PMMA-Knochenzementteigs, zu einer derartigen Erhitzung kommt, dass umliegendes Patientengewebe thermisch geschädigt werden kann. Bei Volumenanteilen von über 70 Volumenprozent an Augmentationsmaterial verringert sich die strukturelle Stabilität des Komposits.

Ein weiterer Gegenstand der Erfindung betrifft ein Verfahren zur Herstellung eines erfindungsgemäßen Komposits umfassend die Schritte
a. Bereitstellen des Augmentationsmaterials in einer Anordnung, welche im Wesentlichen der Form der Kavität entspricht;
b. Applizieren eines Knochenzementteigs in Zwischenräume des Augmentationsmaterials, so dass das Augmentationsmaterial vollständig von dem Knochenzementteig umschlossen ist; und
c. Aushärten des Knochenzementteigs unter Ausbildung des Komposits.

In einem Schritt des Verfahrens erfolgt das Bereitstellen des Augmentationsmaterials. Das Bereitstellen des Augmentationsmaterials in einer Anordnung, welche im Wesentlichen der Form der Kavität entspricht, kann auf unterschiedliche Weise ausgeführt werden. Beispielsweise kann das Augmentationsmaterial spiralförmig um einen Implantatschaft eines Implantats, beispielsweise einer Hüftgelenkendoprothese, gewickelt werden, welches in einen Knochenkanal eingesetzt werden soll. Das Augmentationsmaterial formt dabei im Wesentlichen die Kontur des entsprechenden Knochenkanals nach und dient zum Füllen des Zwischenraums zwischen Implantat und der Wandung des Knochenkanals. Das Augmentationsmaterial kann dabei so angeordnet werden, dass dessen Gruppen an Verbindungselementen form- und/oder kraftschlüssig miteinander verbunden werden. Dies vereinfacht das Bereitstellen des Augmentationsmaterial, da beispielsweise die Stabilität des geformten Körpers aus dem Augmentationsmaterial erhöht wird. Überschüssiges, nicht benötigtes Augmentationsmaterial lässt sich einfach durch Kürzen des Drahtes, beispielsweise mit einer Schere oder einer Zange, entfernen. Sollte der eingesetzte Draht des Augmentationsmaterials nicht ausreichend lang sein, kann ein weiteres Augmentationsmaterial durch Gegeneinanderpressen der jeweiligen Verbindungselemente schnell und unkompliziert mit dem ursprünglichen Draht verbunden werden.

Beim Bereitstellen des Augmentationsmaterials im Wesentlichen in Form der Kavität formt dieses einen stabilen porösen Körper aus.

Die Zwischenräume des Augmentationsmaterials, also die Räume zwischen dem geformten Draht, den ungebunden und miteinander verbundenen Verbindungselementen, werden in einem weiteren Schritt durch Applizieren eines Knochenzementteigs, insbesondere eines PMMA-Knochenzementteig, derart gefüllt, dass das Augmentationsmaterial im Wesentlichen vollständig von dem Knochenzementteig umschlossen ist. Der aus dem Augmentationsmaterial geformte poröse Körper ist im Wesentlichen vollständig mit Knochenzementteig gefüllt und von diesem umschlossen.

In einem weiteren Schritt des Verfahrens erfolgt das Aushärten des Knochenzementteigs unter Ausbildung des Komposits.

Das Verfahren kann vollständig außerhalb der zu befüllenden Kavität durchgeführt werden.

In einer nicht erfindungsgemäßen Ausgestaltungsform des Verfahrens wird das Augmentationsmaterial in der zu befüllenden Kavität bereitgestellt und sowohl das Applizieren des Knochenzementteigs in die Zwischenräume des Augmentationsmaterials sowie das Aushärten des Knochenzementteigs erfolgt in der zu befüllenden Kavität.

Beispielsweise wird das Augmentationsmaterial in einem Knochenkanal bereitgestellt, indem dieses spiralförmig der Kontur des Knochenkanals folgend gewickelt wird. Dabei werden vorzugsweise Gruppen an Verbindungselementen gegeneinandergepresst, um einen stabilen porösen Körper, insbesondere in Form eines Hohlzylinders, auszubilden. In einem weiteren Schritt wird dieser poröse Körper innerhalb des Knochenkanals mit einem Knochenzementteig befüllt, so dass der poröse Körper im Wesentlichen vollständig vom Knochenzementteig umschlossen ist. Bevor der Knochenzementteig aushärtet, wird ein entsprechendes Implantat so in den Knochenkanal eingeschoben, dass ein Schaft des Implantats vom Augmentationsmaterial umgeben ist. Anschließend erfolgt das Aushärten des Knochenzementteigs unter Ausbildung des Komposits.

Die für das Augmentationsmaterial offenbarten Merkmale und Merkmalskombinationen sind auch für das Komposit und das Verfahren offenbart und umgekehrt.

### Figuren

Die Erfindung wird im Folgenden durch Figuren weiter beispielhaft illustriert. Die Erfindung ist nicht auf die Figuren beschränkt.

Es zeigen
- Fig. 1: eine schematische Aufsicht auf einen Abschnitt eines Augmentationsmaterials mit einer Vielzahl von Gruppen an Verbindungselementen,
- Fig. 2: das Augmentationsmaterial aus Figur 1 in einer perspektiven Seitenansicht,
- Fig. 3: eine weitere schematische Aufsicht auf das Augmentationsmaterial aus den Figuren 1 und 2 mit verbundenen Gruppen von Verbindungselementen,
- Fig. 4: eine weitere Ausführungsform eines Augmentationsmaterials in einer schematischen Aufsicht,
- Fig. 5: das Augmentationsmaterial aus Figur 4 in einer perspektiven Seitenansicht,
- Fig. 6: eine weitere Ausführungsform eines Augmentationsmaterials in einer schematischen Aufsicht,
- Fig. 7: das Augmentationsmaterial aus Figur 6 in einer perspektiven Seitenansicht,
- Fig. 8: eine weitere schematische Aufsicht auf das Augmentationsmaterial aus den Figuren 6 und 7 mit verbundenen Gruppen von Verbindungselementen,
- Fig. 9: eine nicht erfindungsgemäße Ausführungsform eines Augmentationsmaterials in einer perspektivischen Seitenansicht,
- Fig. 10: eine nicht erfindungsgemäße Ausführungsform eines Augmentationsmaterials in einer perspektivischen Seitenansicht,
- Fig. 11: eine weitere Ausführungsform eines Augmentationsmaterials in einer perspektivischen Seitenansicht, und
- Fig. 12: ein Flussdiagramm eines Verfahrens zur Herstellung eines Komposits.

### Beschreibung der Figuren

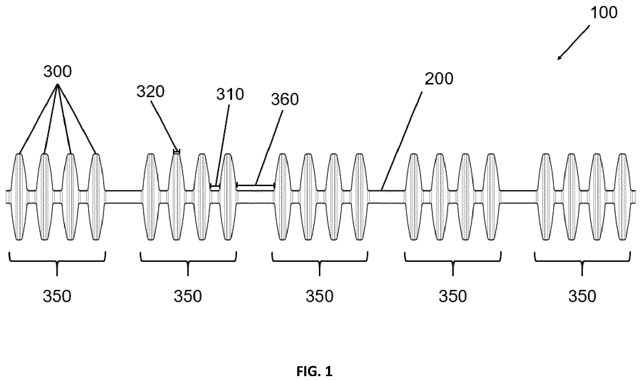

**Figur 1** zeigt eine schematische Aufsicht auf einen Abschnitt eines Augmentationsmaterials 100. Das Augmentationsmaterial 100 umfasst einen Draht 200 und entlang der Längsachse des Drahtes 200 eine Vielzahl an Gruppen 350 von sich radial aus dem Draht 200 erstreckenden und axial benachbarten Verbindungselementen 300 (lediglich exemplarisch mit Bezugszeichen versehen), wobei in Figur 1 lediglich fünf der Gruppen 350 gezeigt sind. Jeder der Gruppen 350 umfasst vier axial benachbarte Verbindungselemente 300 in Form von runden, diskusartig geformten Scheiben, welche konzentrisch um den Draht 200 angeordnet sind. Der Draht 200 und die Verbindungselemente 300 sind einteilig ausgestaltet. Innerhalb einer Gruppe 350 weisen die axial benachbarten Verbindungselemente 300 einen axialen Verbindungselementabstand 310 (lediglich exemplarisch mit Bezugszeichen versehen) auf, welcher ungefähr dem eineinhalbfachen einer axialen Verbindungselementerstreckung 320 (lediglich exemplarisch mit Bezugszeichen versehen) entspricht. Der

Verbindungselementabstand 310 entspricht dabei dem Abstand zweier axial benachbarter Verbindungselemente 300 einer Gruppe 350 auf Höhe des Drahtes 200 und damit der Wegstrecke des Drahtes 200 zwischen diesen Verbindungselementen 300. Die axiale Verbindungselementerstreckung 320 entspricht der axialen Erstreckung der Verbindungselemente 300 an dem dem Draht 200 radial abgewandten Ende der Verbindungselemente 300.

Die einzelnen Gruppen 350 weisen einen axialen Gruppenabstand 360 (lediglich exemplarisch mit Bezugszeichen versehen) zueinander auf, welcher dem dreifachen des Verbindungselementabstands 310. Der axiale Gruppenabstand 360 entspricht, analog dem axialen Verbindungselementabstand 310, dem Abstand zweier axial benachbarter Gruppen 350 auf Höhe des Drahtes und damit der Wegstrecke des Drahtes 200 zwischen diesen Gruppen 350. Der axiale Gruppenabstand 360 ermöglicht eine hohe Flexibilität der Augmentationsvorrichtung 100 und erlaubt das Formen eines porösen Körpers aus dem Augmentationsmaterial 100.

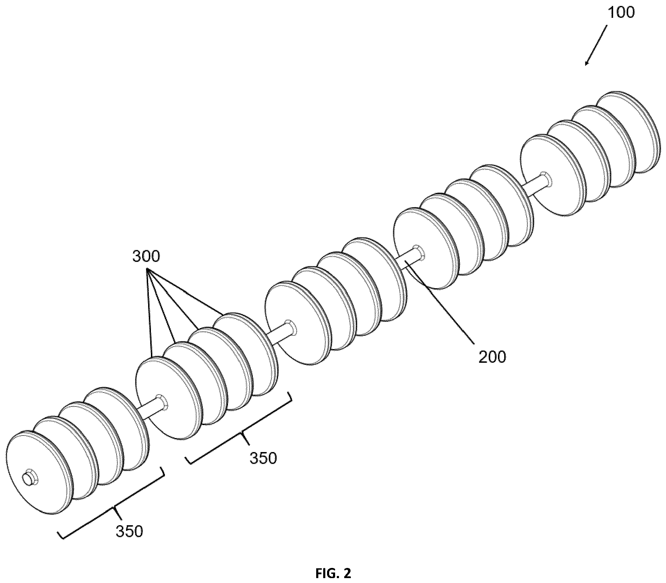

**Figur 2** zeigt den Abschnitt des Augmentationsmaterials 100 aus Figur 1 in einer perspektivischen Seitenansicht.

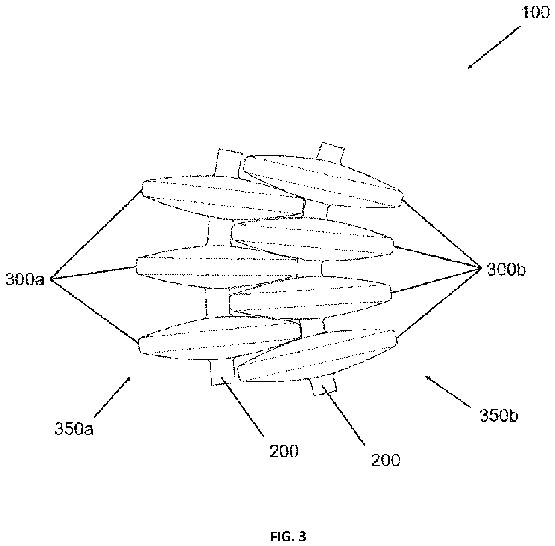

**Figur 3** zeigt eine weitere schematische Aufsicht auf einen Abschnitt des Augmentationsmaterials 100 aus den Figuren 1 und 2, wobei das Augmentationsmaterial 100 derart geformt ist, insbesondere durch ein Verformen des Drahtes 200, dass eine erste Gruppe 350a von Verbindungselementen 300a mit einer zweiten Gruppe 350b von Verbindungselementen 300b des Augmentationsmaterials 100 form- und kraftschlüssig miteinander verbunden ist.

Durch die Abstimmung von Verbindungselementabstand 310 (vgl. Figur 1) zu Verbindungselementerstreckung 320 (vgl. Figur 1) wird ein Formschluss der Verbindungselemente 300a, 300b der beiden Gruppen 350a, 350b erlaubt.

Das Verbinden der beiden gezeigten Gruppen 350a, 350b sowie weiterer, nicht gezeigter Gruppen ermöglicht die Ausbildung eines porösen Körpers unter Verwendung des

Augmentationsmaterials 100. Dieser derart geformte poröse Körper aus dem Augmentationsmaterial 100 kann zur Befüllung von Hohlräumen, insbesondere von Knochenkanälen, und zur Ausbildung eines Komposits aus dem Augmentationsmaterial 100 und einem Knochenzement, insbesondere einem PMMA-Knochenzement, verwendet werden.

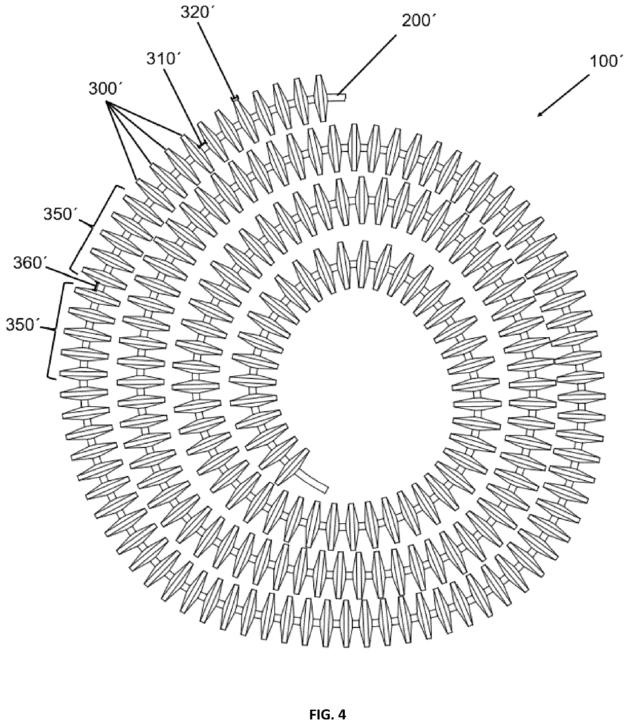

**Figur 4** zeigt eine schematische Aufsicht auf einen Abschnitt einer weiteren Ausführungsform eines Augmentationsmaterials 100'. Die Ausführungsform des Augmentationsmaterials 100' stimmt weitgehend mit den vorstehend beschriebenen und in den Figuren 1 bis 3 dargestellten Ausführungsform überein, so dass zur Vermeidung von Wiederholungen auf die vorstehende Beschreibung verwiesen wird. Abwandlungen einer gegenüber der in den Figuren 1 bis 3 gezeigten Ausführungsform weisen dasselbe Bezugszeichen mit einem zusätzlichen Apostroph auf.

Der axiale Gruppenabstand 360' (lediglich exemplarisch mit Bezugszeichen versehen) der Gruppen 350' (lediglich exemplarisch mit Bezugszeichen versehen) von Verbindungselementen 300' (lediglich exemplarisch mit Bezugszeichen versehen) des Augmentationsmaterials 100' entspricht dem axialen Verbindungselementabstand 310' (lediglich exemplarisch mit Bezugszeichen versehen) von Verbindungselementen 300' innerhalb einer Gruppe 350', so dass die Gruppen 350' wie eine einzige, große Gruppe von Verbindungselementen 300' erscheinen.

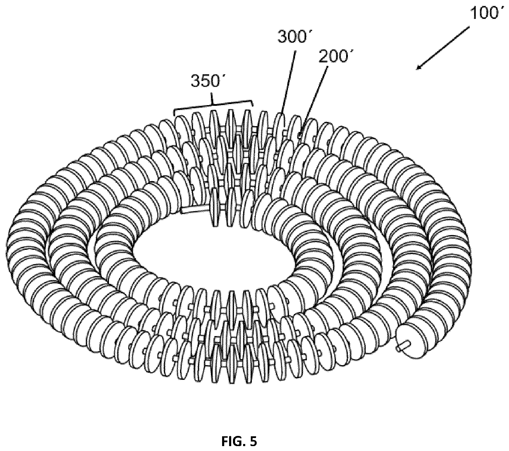

**Figur 5** zeigt das Augmentationsmaterial 100' aus Figur 4 in einer perspektivischen Seitenansicht.

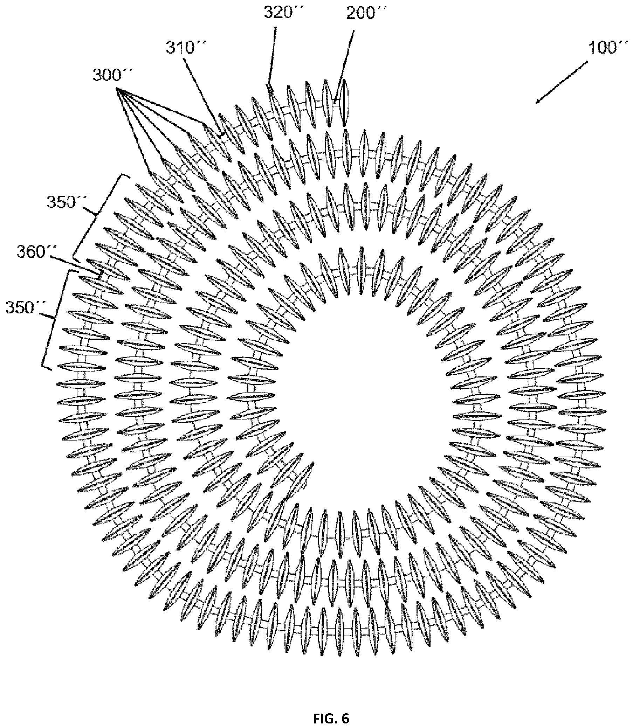

**Figur 6** zeigt eine schematische Aufsicht auf einen Abschnitt einer weiteren Ausführungsform eines Augmentationsmaterials 100". Die Ausführungsform des Augmentationsmaterials 100'' stimmt weitgehend mit den vorstehend beschriebenen und in den Figuren 1 bis 5 dargestellten Ausführungsformen überein, so dass zur Vermeidung von Wiederholungen auf die vorstehende Beschreibung verwiesen wird. Abwandlungen einer gegenüber der in den Figuren 1 bis 5 gezeigten Ausführungsformen weisen dasselbe Bezugszeichen mit zwei Apostrophen auf.

Im Vergleich zu der Ausführungsform des Augmentationsmaterials 100' gemäß den Figuren 4 und 5 weist die in Figur 6 gezeigte Ausführungsform eine geringere axiale Verbindungselementerstreckung 320" (lediglich exemplarisch mit Bezugszeichen versehen) der einzelnen Verbindungselemente 320" auf.

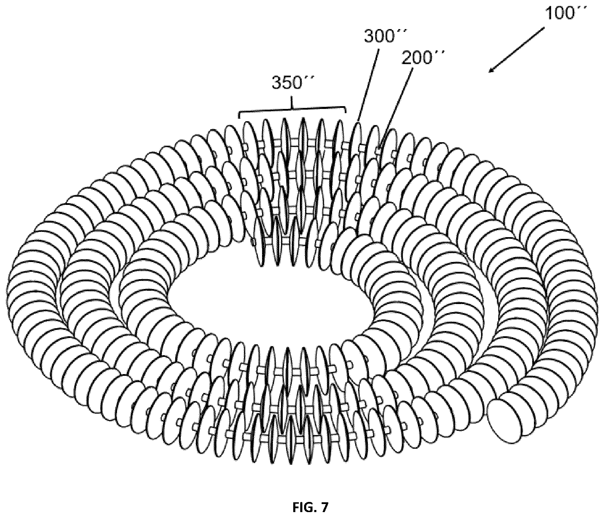

**Figur 7** zeigt das Augmentationsmaterial 100'' aus Figur 6 in einer perspektivischen Seitenansicht.

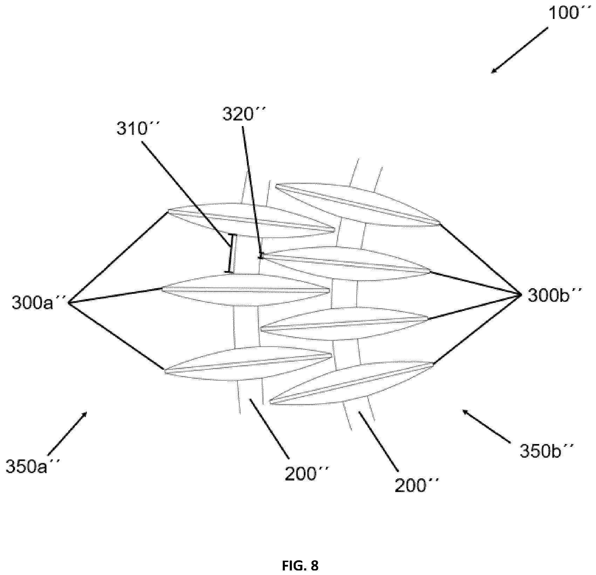

**Figur 8** zeigt eine weitere schematische Aufsicht auf einen Abschnitt des Augmentationsmaterials 100" aus den Figuren 6 und 7, wobei das Augmentationsmaterial 100" derart geformt ist, insbesondere durch ein Verformen des Drahtes 200", dass eine erste Gruppe 350a" von Verbindungselementen 300a" in eine zweiten Gruppe 350b" von Verbindungselementen 300b" des Augmentationsmaterials 100'' ineinandergreift, um die Gruppen 350a'', 350b" bei einer Verschiebung der Gruppen 350a", 350b"entlang der Längsachse des Drahtes 200'' kraftschlüssig miteinander zu verbinden. Aufgrund der zum axialen Verbindungselementabstand 310" geringen axialen Verbindungselementerstreckung 320" bilden die Gruppen 350a", 350b" keinen oder nur partiell einen Formschluss aus.

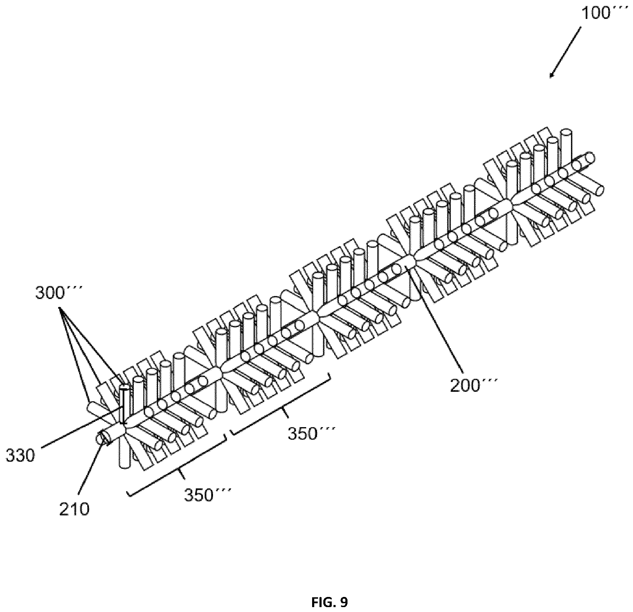

**Figur 9** zeigt eine Aufsicht auf einen Abschnitt einer weiteren, nicht erfindungsgemäßen Ausführungsform eines Augmentationsmaterials 100‴. Die Ausführungsform des Augmentationsmaterials 100‴ stimmt weitgehend mit den vorstehend beschriebenen und in den Figuren 1 bis 8 dargestellten Ausführungsformen überein, so dass zur Vermeidung von Wiederholungen auf die vorstehende Beschreibung verwiesen wird. Abwandlungen einer gegenüber der in den Figuren 1 bis 8 gezeigten Ausführungsformen weisen dasselbe Bezugszeichen mit drei Apostrophen auf.

Die Verbindungselemente 300‴ (lediglich exemplarisch mit Bezugszeichen versehen) der Ausführungsform nach Figur 9 sind in Form von Stiften ausgeformt und nicht wie bei den vorstehenden Ausführungsformen nach den Figuren 1 bis 8 in Form von Scheiben. Jeder der axial benachbarten Gruppen 350‴ (lediglich exemplarisch mit Bezugszeichen versehen) von Verbindungselementen 300‴ in Form von Stiften weist fünf axial benachbarte Stifte auf, wobei jeder dieser Stifte weitere sieben radial benachbarte Stifte aufweist. Jeder der Gruppen 350‴ weist somit insgesamt 40 Verbindungselemente 300‴ in Form von Stiften auf. Eine Gruppe 350‴ umfasst somit jeweils fünf Untergruppen von jeweils acht radial benachbarten Verbindungselementen 300‴ in Form von Stiften, wobei die Untergruppen von jeweils acht radial benachbarten Stiften sich in einer Querschnittsebene des Drahtes 200‴ radial aus dem Draht 200‴ erstrecken. Um die Verbindungselemente 300‴ in Form von Stiften zweier Gruppen 350‴ effektiv kraftschlüssig miteinander verbinden zu können, weisen die Stifte eine Stiftlänge 330 auf, welche dem dreifachen eines Drahtdurchmessers 210 des Drahtes 200‴ entspricht. Die einzelnen Gruppen 350" sind analog zu dem Augmentationsmaterial 100 der Figuren 1 bis 3 zueinander beabstandet.

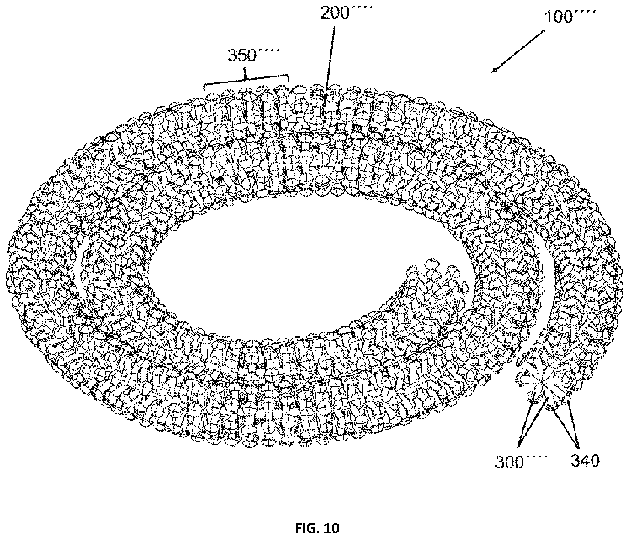

**Figur 10** zeigt eine perspektivische Seitenansicht einer weiteren, nicht erfindungsgemäßen Ausführungsform eines Augmentationsmaterials 10"". Die Ausführungsform des Augmentationsmaterials 100' stimmt weitgehend mit den vorstehend beschriebenen und in den Figuren 1 bis 9 dargestellten Ausführungsformen überein, so dass zur Vermeidung von Wiederholungen auf die vorstehende Beschreibung verwiesen wird. Abwandlungen einer gegenüber der in den Figuren 1 bis 9 gezeigten Ausführungsformen weisen dasselbe Bezugszeichen mit vier Apostrophen auf. Die Verbindungselemente 300‴ (lediglich exemplarisch mit Bezugszeichen versehen) der Ausführungsform nach Figur 10 sind in Form von Stiften, ähnlich zu den Verbindungselementen gemäß der Ausführungsform nach Figur 9, ausgeformt. Die Verbindungselemente 300"" in Form von Stiften weisen an einem dem Draht entgegengesetzten Ende jeweils einen Pilz 340 auf. Die Pilze 340 rasten beim Gegeneinanderpressen zweier Gruppen 350"" von Verbindungselementen 300"" miteinander, so dass das Augmentationsmaterial 100"" einen sehr stabilen porösen Körper ausformen kann.

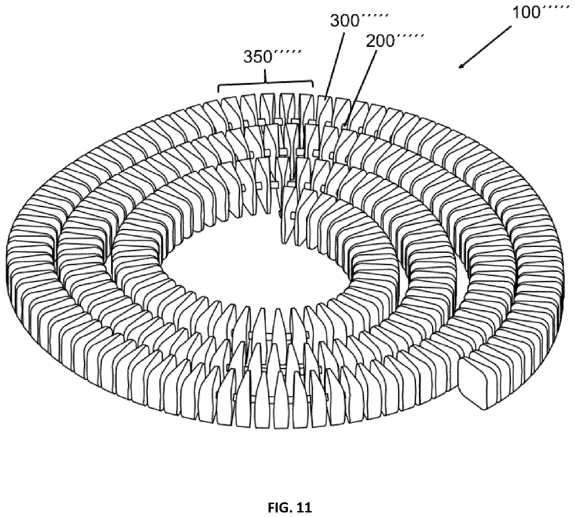

**Figur 11** zeigt eine perspektivische Seitenansicht einer weiteren Ausführungsform eines Augmentationsmaterials 10"‴. Die Ausführungsform des Augmentationsmaterials 100‴" stimmt weitgehend mit den vorstehend beschriebenen und in den Figuren 1 bis 10 dargestellten Ausführungsformen überein, so dass zur Vermeidung von Wiederholungen auf die vorstehende Beschreibung verwiesen wird. Abwandlungen einer gegenüber der in den Figuren 1 bis 10 gezeigten Ausführungsformen weisen dasselbe Bezugszeichen mit fünf Apostrophen auf. Die Verbindungselemente 300"‴ sind als keilförmige Scheiben ausgestaltet, so dass eine gute kraftschlüssige Verbindung der Gruppen 350"‴ beim Gegeneinanderpressen ermöglicht wird.

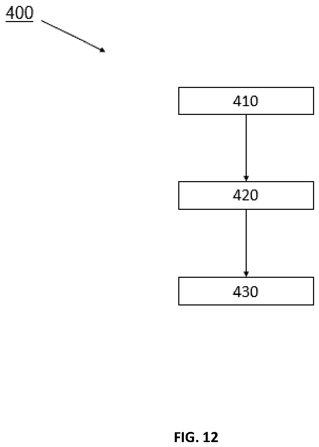

**Figur 12** zeigt ein Flussdiagramm eines Verfahrens 400 zur Herstellung eines Komposits umfassend das Augmentationsmaterial 100, 100', 100", 100‴, 100"", 100‴" und einen Knochenzement mit den Schritten 410 bis 430. Der Komposit dient dem Befüllen einer Kavität. In Schritt 410 erfolgt ein Bereitstellen des Augmentationsmaterials 100, 100', 100'', 100‴, 100", 100‴" in Form eines porösen Körpers, welcher im Wesentlichen der Form der Kavität entspricht. Dazu kann das Augmentationsmaterial 100, 100', 100", 100‴, 100"", 100‴" gekürzt werden, beispielsweise mittels einer Schere oder Zange, so dass die Form der Kavität möglichst passend nachgebildet werden kann. Zum Ausbilden der Form der Kavität ist es bevorzugt, dass zwei oder mehr der Gruppen 350, 350', 350", 350‴, 350"", 350‴" von Verbindungselementen 300, 300', 300", 300‴, 300"", 300"‴ gegeneinandergepresst werden, so dass diese form- und/oder kraftschlüssig zusammenwirken und so den porösen Körper aus Augmentationsmaterial 100, 100', 100", 100‴, 100"", 100‴" strukturell stabilisieren.

In Schritt 420 wird ein Knochenzementteig in Zwischenräume des aus dem Augmentationsmaterial 100, 100', 100'', 100‴, 100", 100‴" geformten und bereitgestellten porösen Körpers appliziert, so dass die Zwischenräume mit Knochenzementteig gefüllt sind und das Augmentationsmaterial 100, 100', 100", 100‴, 100"", 100‴" von dem Knochenzementteig umschlossen ist.

In Schritt 430 härtet der den porösen Körper aus Augmentationsmaterial 100, 100', 100", 100‴, 100"", 100‴" umschließenden Knochenzementteig unter Ausbildung des Komposits aus. Dabei geht der Knochenzementteig in Knochenzement über.

Der so Komposit hergestellte Komposit in die zu befüllende Kavität, insbesondere einen Knochenkanal, eingesetzt werden.

In einer nicht erfindungsgemäßen Ausgestaltungsform des Verfahrens 400 findet das Bereitstellen 410 des Augmentationsmaterials 100, 100', 100", 100‴, 100"", 100‴" in der zu befüllenden Kavität, insbesondere einem Knochenkanal, statt. Zu dem in der Kavität bereitgestellten Augmentationsmaterial 100, 100', 100'', 100‴, 100", 100‴" in Form eines porösen Körpers wird in Schritt 420 der Knochenzementteig appliziert, so dass die Zwischenräume mit Knochenzementteig gefüllt sind und das Augmentationsmaterial 100, 100', 100", 100‴, 100", 100‴" von dem Knochenzementteig umschlossen ist. Das Aushärten 430 des Knochenzementteigs unter Ausbildung des Komposits erfolgt ebenso in der zu befüllenden Kavität.

### Bezugszeichen

- 100, 100', 100" 100‴, 100"", 100‴": Augmentationsmaterial
- 200, 200', 200" 200"', 200"", 200"‴: Draht
- 210: Drahtdurchmesser
- 300, 300', 300" 300‴, 300"", 300"‴: Verbindungselement
- 300a: Verbindungselemente erste Gruppe
- 300b: Verbindungselemente weiterer Gruppe
- 310, 310', 310": axialer Verbindungselementabstand
- 320, 320', 320": axiale Verbindungselementerstreckung
- 330: Stiftlänge
- 340: Pilz
- 350, 350', 350" 350‴, 350"", 350"‴: Gruppe von Verbindungselementen
- 350a, 350a'': erste Gruppe von Verbindungselementen
- 350b, 350b'': weitere Gruppe von Verbindungselementen
- 360, 360', 360": axialer Gruppenabstand
- 400: Verfahren zur Herstellung eines Komposits
- 410: Bereitstellen
- 420: Applizieren
- 430: Aushärten

## Patentansprüche

1. Augmentationsmaterial (100, 100', 100", 100‴") umfassend
einen Draht (200, 200', 200", 200‴") und in axialer Ausrichtung entlang einer Längsachse des Drahtes (200, 200', 200", 200‴") eine Vielzahl an Gruppen (350, 350', 350", 350‴") von sich radial aus dem Draht (200, 200', 200", 200‴") erstreckenden und axial benachbarten Verbindungselementen (300, 300', 300", 300‴"), wobei die Verbindungselemente (300, 300', 300", 300‴") derart ausgestaltet sind, dass beim Gegeneinanderpressen einer ersten Gruppe (350a, 350") aus der Vielzahl an Gruppen (350, 350', 350", 350‴") gegen eine weitere Gruppe (350b, 350") aus der Vielzahl an Gruppen (350, 350', 350", 350‴") die Verbindungselemente (300a, 300a", 300b, 300b") der beiden Gruppen (350a, 350a" 350b, 350b") form- und/oder kraftschlüssig miteinander verbindbar sind,
**dadurch gekennzeichnet, dass** die Verbindungselemente (300, 300', 300", 300‴") als sich radial aus dem Draht (200, 200', 200'', 200‴") erstreckende Scheiben ausgebildet sind.

2. Augmentationsmaterial (100, 100', 100", 100‴") nach Anspruch 1, wobei axial benachbarte Verbindungselemente (300, 300', 300", 300‴") in einer Gruppe (350) einen axialen Verbindungselementabstand (310, 310', 320") zueinander aufweisen, welcher zumindest einer axialen Verbindungselementerstreckung (320, 320', 320") eines Verbindungselements (300, 300', 300", 300‴") entlang der Längsachse des Drahtes (200, 200', 200", 200‴") entspricht.

3. Augmentationsmaterial (100, 100', 100", 100‴") nach Anspruch 1 oder 2, wobei die Scheiben perforiert sind und/oder eine offenporige Struktur aufweisen.

4. Augmentationsmaterial (100, 100', 100", 100‴") nach einem der vorhergehenden Ansprüche, wobei eine Gruppe (350, 350', 350", 350‴") von Verbindungselementen (300, 300', 300", 300‴") 3 bis 20 axial benachbarter Verbindungselemente (300, 300', 300", 300‴") umfasst.

5. Augmentationsmaterial (100) nach einem der vorhergehenden Ansprüche, wobei axial benachbarte Gruppen (350) von Verbindungselementen (300) einen axialen Gruppenabstand (360) zueinander aufweisen, welcher zumindest dem zweifachen der axialen Erstreckung eines Verbindungselements (300) entlang der Längsachse des Drahtes (200) entspricht.

6. Komposit umfassend ein Augmentationsmaterial (100, 100', 100", 100‴") nach einem der vorhergehenden Ansprüchen 1 bis 5 und einen Knochenzement, insbesondere einen PMMA-Knochenzement, wobei das Augmentationsmaterial (100, 100', 100", 100‴" von dem Knochenzement umschlossen ist.

7. Komposit nach Anspruch 6, wobei das Augmentationsmaterial (100, 100', 100", 100‴") im Komposit einen Volumenanteil im Bereich von 30-70 Volumenprozent bezogen auf das Volumen des Komposits einnimmt.

8. Verfahren (400) zur Herstellung eines Komposits nach einem der Ansprüche 6 oder 7 zum Befüllen einer Kavität umfassend die Schritte
a. Bereitstellen (410) des Augmentationsmaterials (100, 100', 100", 100‴") in einer Anordnung, welche im Wesentlichen die Kontur der Kavität nachbildet;
b. Applizieren (420) eines Knochenzementteigs in Zwischenräume des Augmentationsmaterials (100, 100', 100", 100‴"), so dass das Augmentationsmaterial (100, 100', 100", 100‴") vollständig von dem Knochenzementteig umschlossen ist; und
c. Aushärten (430) des Knochenzementteigs unter Ausbildung des Komposits.

## Claims

1. An augmentation material (100, 100', 100", 100‴") comprising a wire (200, 200', 200", 200‴") and, in axial alignment along a longitudinal axis of the wire (200, 200', 200", 200""'), a plurality of groups (350, 350', 350", 350‴ʺ) of axially adjacent connecting elements (300, 300', 300", 300‴ʺ) which extend radially from the wire (200, 200', 200", 200‴ʺ), the connecting elements (300, 300', 300", 300‴") being designed such that, when a first group (350a, 350") of the plurality of groups (350, 350', 350", 350‴ʺ) presses against a further group (350b, 350") of the plurality of groups (350, 350', 350", 350‴ʺ), the connecting elements (300a, 300a", 300b, 300b") of the two groups (350a, 350a" 350b, 350b") can be connected to one another in a form-fitting and/or force-fitting manner, **characterized in that** the connecting elements (300, 300', 300", 300‴") are designed as discs which extend radially from the wire (200, 200', 200", 200‴ʺ).

2. The augmentation material (100, 100', 100", 100‴") according to claim 1, wherein axially adjacent connecting elements (300, 300', 300", 300‴ʺ) in a group (350) have an axial connecting element spacing (310, 310', 320") from one another, which spacing corresponds to at least one axial connecting element extension (320, 320', 320") of a connecting element (300, 300', 300", 300‴") along the longitudinal axis of the wire (200, 200', 200", 200‴ʺ).

3. The augmentation material (100, 100', 100", 100""') according to claim 1 or 2, wherein the discs are perforated and/or have an open-pore structure.

4. The augmentation material (100, 100', 100", 100‴") according to any of the preceding claims, wherein a group (350, 350', 350", 350‴") of connecting elements (300, 300', 300", 300‴") comprises 3 to 20 axially adjacent connecting elements (300, 300', 300", 300‴ʺ).

5. The augmentation material (100) according to any of the preceding claims, wherein axially adjacent groups (350) of connecting elements (300) have an axial group spacing (360) from one another, which spacing corresponds to at least twice the axial extension of a connecting element (300) along the longitudinal axis of the wire (200).

6. A composite comprising an augmentation material (100, 100', 100", 100""') according to any of the preceding claims 1 to 5 and a bone cement, in particular a PMMA bone cement, wherein the augmentation material (100, 100', 100", 100‴") is surrounded by the bone cement.

7. The composite according to claim 6, wherein the augmentation material (100, 100', 100", 100""') in the composite occupies a volume fraction in the range of 30-70 percent by volume, based on the volume of the composite.

8. A method (400) for producing a composite according to either of claims 6 or 7 for filling a cavity, comprising the steps of
a. providing (410) the augmentation material (100, 100', 100", 100‴") in an arrangement which substantially follows the contour of the cavity;
b. applying (420) a bone cement paste into gaps in the augmentation material (100, 100', 100", 100‴ʺ), so that the augmentation material (100, 100', 100", 100‴") is completely surrounded by the bone cement paste; and
c. hardening (430) the bone cement paste to form the composite.

## Revendications

1. Matériau d'augmentation (100, 100', 100", 100‴") comprenant un fil (200, 200', 200", 200‴") et, en alignement axial le long d'un axe longitudinal du fil (200, 200', 200", 200‴ʺ), une pluralité de groupes (350, 350', 350", 350‴") d'éléments de liaison (300, 300', 300", 300‴") s'étendant radialement à partir du fil (200, 200', 200", 200‴") et axialement adjacents, dans lequel les éléments de liaison (300, 300', 300", 300‴") sont conçus de telle sorte que, lorsqu'un premier groupe (350a, 350") de la pluralité de groupes (350, 350', 350", 350‴") est pressé contre un autre groupe (350b, 350") de la pluralité de groupes (350, 350', 350", 350‴ʺ), les éléments de liaison (300a, 300a", 300b, 300b") des deux groupes (350a, 350a", 350b, 350b") peuvent être reliés entre eux par complémentarité de forme et/ou par liaison à force,
**caractérisé en ce que** les éléments de liaison (300, 300', 300", 300‴") sont réalisés sous forme de disques s'étendant radialement hors du fil (200, 200', 200", 200‴ʺ).

2. Matériau d'augmentation (100, 100', 100", 100""') selon la revendication 1, dans lequel des éléments de liaison (300, 300', 300", 300‴") axialement adjacents dans un groupe (350) présentent un espacement entre éléments de liaison axial (310, 310', 320") entre eux, lequel espacement correspond au moins à une extension d'élément de liaison axiale (320, 320', 320") d'un élément de liaison (300, 300', 300", 300‴") le long de l'axe longitudinal du fil (200, 200', 200", 200""').

3. Matériau d'augmentation (100, 100', 100", 100‴") selon la revendication 1 ou 2, dans lequel les disques sont perforés et/ou présentent une structure à pores ouverts.

4. Matériau d'augmentation (100, 100', 100", 100""') selon l'une des revendications précédentes, dans lequel un groupe (350, 350', 350", 350‴ʺ) d'éléments de liaison (300, 300', 300", 300‴") comprend 3 à 20 éléments de liaison (300, 300', 300", 300""') axialement adjacents.

5. Matériau d'augmentation (100) selon l'une des revendications précédentes, dans lequel des groupes (350) axialement adjacents d'éléments de liaison (300) présentent entre eux un espacement entre groupes axial (360) qui correspond à au moins deux fois l'extension axiale d'un élément de liaison (300) le long de l'axe longitudinal du fil (200).

6. Composite comprenant un matériau d'augmentation (100, 100', 100", 100‴") selon l'une des revendications précédentes 1 à 5 et un ciment osseux, en particulier un ciment osseux PMMA, dans lequel le matériau d'augmentation (100, 100', 100", 100""') est entouré par le ciment osseux.

7. Composite selon la revendication 6, dans lequel le matériau d'augmentation (100, 100', 100", 100""') occupe dans le composite une fraction volumique dans la plage allant de 30 à 70 pour cent en volume par rapport au volume du composite.

8. Procédé (400) destiné à la fabrication d'un composite selon l'une des revendications 6 ou 7 pour le remplissage d'une cavité comprenant les étapes consistant à
a. fournir (410) le matériau d'augmentation (100, 100', 100", 100""') dans un agencement qui reproduit sensiblement le contour de la cavité ;
b. appliquer (420) une pâte de ciment osseux dans des interstices du matériau d'augmentation (100, 100', 100", 100‴") de telle sorte que le matériau d'augmentation (100, 100', 100", 100‴") est complètement entouré par la pâte de ciment osseux ; et
c. durcir (430) la pâte de ciment osseux pour former le composite.
